# EUROPEAN PATENT SPECIFICATION

(11) **EP 3 174 558 B1**
(45) Date of publication and mention of the grant of the patent: **06.03.2019**
(21) Application number: 15738943.8
(22) Date of filing: 22.07.2015
(51) Int. Cl.: A61K 49/10, A61K 49/18

(54) **PREPARATION OF AMORPHOUS SAMPLES FOR DNP FROM SOLID CRYSTALLINE SUBSTRATES**
HERSTELLUNG AMORPHER PROBEN FÜR DNP AUS FESTEN KRISTALLINEN SUBSTRATEN
PRÉPARATION D'ÉCHANTILLONS AMORPHES POUR DNP À PARTIR DE SUBSTRATS CRISTALLINS SOLIDES

(30) Priority: 29.07.2014 EP 14178980
(43) Date of publication of application: 07.06.2017
(73) Proprietor: Bracco Imaging S.p.A., 20134 Milano (IT); Universita di Pavia, 27100 Pavia (IT)
(72) Inventor: ELISEI, Elena, 34127 Trieste (IT); CESARO, Attilio, 34127 Trieste (IT); FILIBIAN, Marta, 27100 Pavia (IT); CARRETTA, Pietro, 27100 Pavia (IT); COLOMBO SERRA, Sonia, 10010 Colleretto Giacosa (IT); MAIOCCHI, Alessandro, 10010 Colleretto Giacosa (IT); TEDOLDI, Fabio, 10010 Colleretto Giacosa (IT)
(74) Representative: Ravizza, Claudio
(86) International application number: PCT/EP2015/066783
(87) International publication number: WO 2016/016066

(56) References cited:
- WO-A2-2007/136439
- US-A1- 2010 092 396
- US-A1- 2011 062 392
- KAYVAN R. KESHARI ET AL: "Chemistry and biochemistry of 13C hyperpolarized magnetic resonance using dynamic nuclear polarization", CHEMICAL SOCIETY REVIEWS, vol. 43, no. 5, 1 January 2014 (2014-01-01), page 1627, XP055162550, ISSN: 0306-0012, DOI: 10.1039/c3cs60124b
- TA-CHUNG ONG ET AL: "Solvent-Free Dynamic Nuclear Polarization of Amorphous and Crystalline ortho -Terphenyl", THE JOURNAL OF PHYSICAL CHEMISTRY B, vol. 117, no. 10, 14 March 2013 (2013-03-14), pages 3040-3046, XP055162510, ISSN: 1520-6106, DOI: 10.1021/jp311237d

## Description

### FIELD OF THE INVENTION

The present invention relates to the field of Dynamic Nuclear Polarization (DNP). In particular, it relates to a method of preparation of solid amorphous substrates for DNP. The invention relates to a method for the manufacture of a sample in amorphous form for dynamic nuclear polarization which comprises submitting a solid substrate in a crystalline form to a milling process in the presence of a polarizing agent at a temperature lower than the glass-transition temperature (Tg) of said substrate.

### BACKGROUND OF THE INVENTION

Dynamic nuclear polarization (DNP) is often used in magnetic resonance spectroscopy (MRS) to enhance the NMR signal of a sample comprising NMR active nuclei. Polarization of the sample is effected through a polarizing agent prior to its administration and MR signal measurement.

The term "hyperpolarization" means enhancing the nuclear polarization of the NMR active nuclei present in the agent, i.e. nuclei with non-zero nuclear spin, preferably ¹³Cor ¹⁵N-nuclei, and thereby amplifying the MR signal intensity by a factor of hundred and more. When using a hyperpolarized ¹³C- and/or ¹⁵N-enriched high T₁ agent, there will be essentially no interference from background signals as the natural abundance of ¹³C and/or ¹⁵N is negligible and thus the image contrast will be advantageously high. The main difference between conventional MRI contrast agents and these hyperpolarized high T₁ agents is that in the former changes in contrast are caused by affecting the relaxation times of water protons in the body whereas the latter class of agents can be considered as non-radioactive tracers, as the signal obtained arises solely from the agent. When hyperpolarization is obtained via a microwave assisted transfer between unpaired electrons and the nuclei used as MR probes, the technique is referred as Dynamic Nuclear Polarization.

For example, US6278893 and US6466814 disclose methods of magnetic resonance imaging using ex vivo polarization of an MR imaging agent.

In the dynamic nuclear polarization (DNP) technique, polarization of the sample is effected by a paramagnetic compound, the so-called polarizing agent (in particular a radical compound), which shall be intimately and homogeneously admixed with the sample to be polarized. During the DNP process, energy is provided, normally in the form of microwave radiation, which will initially excite the polarizing agent. Upon decay to the ground state, there is a transfer of polarization from the unpaired electron of polarizing agent to the NMR active nuclei of the sample. Typically, a moderate or high magnetic field and very low temperatures (e.g. 1 to 3°K) are used in the DNP process, e.g. by carrying out the DNP process in liquid helium and a magnetic field of about 1 T or above. Alternatively, a moderate magnetic field and any temperature at which sufficient polarization enhancement is achieved may be employed. The DNP technique is for example described in WO98/58272 and in WO01/96895.

The substrate to be polarized by DNP can be in liquid or solid form at room temperature.

When the substrate is liquid at room temperature, the DNP polarizing agent (usually a radical) is preferably capable of being dissolved in the liquid sample, so as to avoid the need of further solvents being present in the mixture.

When the substrate is in solid form, the intimate mixing with the polarizing agent is achieved either by dissolving the sample in a suitable solvent or by melting the sample in the presence of the polarizing agent.

The obtained liquid mixture is then typically subjected to a sudden freezing (flash-freezing) down to the desired temperature for performing the DNP process so that a solid frozen mixture is obtained. The flash-freezing step has usually also the consequence of maintaining the mixture in an amorphous state.

Whichever the technique to obtain the solid frozen mixture, in order to effectively perform the DNP process the final solid sample shall be in an amorphous state, as an effective polarization cannot be achieved on crystalline solids.

For amorphous state it is intended that the solid is not in crystalline form, thus a crystalline network is not present in the solid.

For the DNP process to be effective, the radicals must also be homogeneously distributed within the amorphous sample.

As many substrates of diagnostic interest are crystalline solids at room temperature, methods have been developed for converting these compounds into an amorphous form and at the same time provide the necessary intimate mixing with the polarizing agent. These methods typically include:
1. dissolution of the crystalline sample in a liquid glass forming agent (such as water/glycerol solution or DMSO) (1, 2, 3, 4, 5); or
2. melting of the sample (5,6);

Typically, the radical is admixed with the substrate either before or after the dissolution/melting of the sample and the mixture is then subjected to flash-freezing (to the desired temperature for the DNP process) to obtain the desired amorphous sample.

In the first method, the solid compound is dissolved in an adequate solvent or solvent mixture which is a good glass former and does prevent crystallization upon cooling/freezing. The dissolved compound is then mixed with the radical and the solution is cooled and/or frozen for the DNP process.

For example, in WO2004092759 radicals to use for the DNP of a sample are disclosed. The use of glass forming compounds is recommended to ensure the homogeneous distribution of radicals and sample in the frozen mixture, which is important to achieve a high DNP effect.

Also in WO2008086534, a glass-former is added to prevent crystallization and to produce an amorphous solid after cooling the sample.

However, this method has several disadvantages. First of all, vitrifying agents increase sample volumes thus leading to a decrease of substrate concentration; also, the current design of the clinical polarization set-up imposes constraints on the sample amount to be polarized since cooling, radiofrequency/microwave irradiation and dissolution are less efficient when sample volumes are large. Secondly, the addition of a glass forming agent may introduce toxicology issues and metabolic interferences in case the product is intended for medical use. For example, some vitrifying agents can be metabolized themselves and may thus distort metabolic *in vivo* assays. Also, vitrification is still a challenge, due to the lack of fine control on the freezing step and on the behavioural variety depending on the solvent as well as the solute nature and concentration.

In the second method, the solid compound is heated up to the melting temperature and then mixed with the radical to dissolve the radical in the molten compound. Subsequently, the solution is cooled and/or frozen, generally in such a way that crystallization of the compound to be polarized is prohibited.

Also this second method presents some disadvantages. First of all, since it involves a heating step it can lead to the degradation of the radical and/or of the sample; besides, the passages of melting and subsequent cooling/freezing need to be carefully monitored and performed in order to assure firstly a complete melting of the sample preventing at the same time its degradation and secondly a cooling/freezing step rapid enough to prevent recrystallization of the compound. These disadvantages of the method limit its industrial use, wherein a "ready to use" formulation, i.e. a sample formulation allowing its immediate polarization without further complicated passages, is highly preferred.

Both the above methods are described, for example, in WO2006011811 disclosing certain radicals to use as polarizing agents in DNP.

Therefore, there is still the need for a method for obtaining a sample for use in DNP from compounds which are solid at room temperature, which may avoid at least partially the above mentioned disadvantages of the methods known in the art.

In particular, many small organic molecules, which are particularly useful as a substrate for metabolic DNP applications, are crystalline solids at room temperature. Therefore, a method for obtaining solid amorphous samples ready for DNP from this kind of substances is highly desired, WO-A-2007/136439 discloses methods and systems for providing hyperpolarized materials. In an embodiment, a powder may be hyperpolarized, which may be provided from a material that is solid at standard conditions, using a technique such as grinding, attrition mills, or plasma sputtering techniques.

The inventors of the present invention have now found a new method for the preparation of a solid amorphous sample for DNP of a solid compound comprising the milling of the solid compound to be polarized (in short "the substrate") with a polarizing agent (in short "the radical").

Surprisingly, this new method allows obtaining a solid sample of the substrate in an amorphous form with an intimate and even dispersion of the radical into the amorphous structure of the sample.

While the method of the invention is applied to starting compounds that are crystalline solid (and thus may undergo amorphization during the milling process), the method may, according to the disclosure, also be used with starting compounds that are amorphous solid and may thus be subjected to co-milling with the radical in order to obtain the desired intimate and even dispersion of the radical into the amorphous sample.

Use of mechanical milling is known in the preparation of pharmaceutical formulation.

Indeed, mechanical milling is used in the pharmaceutical field to increase drug solubility by changing the physical state of the material, for example reducing particles size or through an amorphization process (7).

However, milling has never been used in the preparation of samples for DNP procedures.

Surprisingly, the use of milling for the preparation of samples for DNP allows obtaining a mixture of the solid substrate (in amorphous form) with the radical compound, wherein the radical compound is intimately and evenly dispersed into the amorphous structure of the sample thus allowing an effective interaction at molecular level between the radical and the compound to be polarized, so to efficiently perform the subsequent polarization process.

Also, the obtained intimate and homogenous mixture of the substrate with the polarizing agent in the solid sample is stable and the sample can thus be stored (optionally at low temperatures, typically lower than substrate's Tg) until needed.

An advantage with respect to the known methods is that the method of the invention does neither require the use of any solvent for dissolving the solid substrate and admixing it with the polarizing agent (thus avoiding possible problems such as decrease of substrate concentration, possible presence of potentially toxic compounds or metabolic interferences) nor does it require a heating step in order to allow its mixing with the polarizing agent in the molten state (thus avoiding possible related problems such as degradation or alteration of the radical or of the sample).

A further advantage is that the mixture obtained with the method of the invention is ready to be cooled/frozen for use in the DNP process without the need of further critical passages therefore providing a "ready to use" formulation which is highly desired, especially in industrial applications.

A still further advantage relies on the possibility of analysing and characterizing the obtained solid amorphous structure before the DNP process takes place. With the existing methods the glassy amorphous structure is reached by a flash freezing process within the helium bath where the sample is introduced just before starting the microwave irradiation inducing the DNP. Therefore the amorphization of the sample cannot be monitored or checked before polarization, so that the degree of crystallinity, which is a fundamental parameter affecting the polarization performances, is not under control. With the method of the invention, instead, the amorphous structure is obtained well before the DNP process thus allowing analysis and monitoring of the crystallinity degree of the sample.

### SUMMARY OF THE INVENTION

It is an object of the present invention to provide a method for the manufacture of a sample in amorphous form for dynamic nuclear polarization which comprises submitting a solid substrate in a crystalline form to a milling process in the presence of a polarizing agent at a temperature lower than the glass-transition temperature (Tg) of said substrate.

In a preferred embodiment the solid substrate in crystalline form is admixed with the polarizing agent and the obtained mixture is submitted to the milling process.

In an alternative embodiment the solid substrate in crystalline form is submitted to a first milling process; the polarizing agent is then admixed to the milled substrate and the mixture is submitted to a second milling process.

Typically the milling process is performed at a temperature lower than the Tg of the substrate, Preferably, the method of the invention is performed at a temperature at least 10°C lower than the Tg, more preferably at a temperature at least 30°C lower and even more preferably at a temperature at least 50°C lower than the Tg.

In particular, when said substrate has a glass-transition temperature (Tg) well above room temperature (e.g. Tg > 35°C), the method of the invention is preferably carried out at room temperature. Otherwise, when the Tg of the substrate is closer to room temperature, the system for performing the milling process preferably undergoes cooling, in order to perform the process at the desired temperature lower than the Tg.

A further aspect of the invention relates to a method for preparing a polarized sample comprising a substrate polarized by dynamic nuclear polarization (DNP) which comprises the following steps:
a) submitting the solid substrate in a crystalline form to a milling process in the presence of a polarizing agent to obtain a sample comprising said substrate in amorphous form and said polarizing agent;
b) submitting said sample to DNP to obtain a polarized sample comprising said polarized substrate.

A still further aspect relates to a method for preparing a solution for MRI analysis which comprises the following steps:
a) preparing a polarized sample as above defined, and
b) dissolving said polarized sample in an appropriate solvent.

According to another aspect, the disclosure relates to a method of MR analysis which comprises the following steps:
a) preparing a solution for MR analysis as described above;
b) administering said solution to a subject in need thereof; and
c) detecting a MR signal from the polarized substrate and/or from a metabolic product thereof.

According to another aspect, the disclosure relates to a method of MR analysis on a subject who has been previously administered with a solution for MR analysis comprising a polarized substrate prepared as described above which comprises the step of detecting a MR signal from said polarized substrate and/or from a metabolic product thereof.

### Figures

Figure 1: Raman spectrum of crystalline β-Trehalose.
Figure 2: Calorimetric thermogram of crystalline β-Trehalose.
Figure 3: Raman spectrum of a milled sample of Trehalose and TEMPO.
Figure 4: Calorimetric thermogram of a milled sample of Trehalose and TEMPO.
Figure 5: Calorimetric thermogram of a milled sample of Lactose and TEMPO.
Figure 6: Calorimetric thermogram of a milled sample of Lactose, Glucose and TEMPO.
Figure 7: Thermogravimetric curve of the milled sample of Lactose.
Figure 8: Enhancement ε of the ¹H NMR signal under MW irradiation at 96.93 GHZ at 1.7 K as a function of the magnetic field in the milled sample of Trehalose with radical concentration of 0.64%.
Figure 9: Polarization of ¹H under MW irradiation around 96.93 GHz at 4.2 K (diamonds) and 1.75 K (squares) as a function of the radical concentration in milled samples of Trehalose expressed as weight fraction.
Fig. 10: ¹H polarization build-up in milled Lactose+Glucose with radical concentration of 0.60% with MW irradiation (full dots) and without MW irradiation (empty dots).

### Definitions

Within the scope of the present invention, the terms "hyperpolarization", "hyperpolarized" or similar mean enhancing the nuclear polarization of NMR active nuclei present in the target agent as commonly intended in the state of the art. The term "hyperpolarized" refers in particular to the nuclear spin polarization of a compound higher than thermal equilibrium.

As used herein, the term Dynamic Nuclear Polarization (DNP) refers to a technique for (hyper)polarizing a substrate, which comprises submitting the substrate in the presence of a polarizing agent to a microwave irradiation at very low temperatures as commonly intended in the state of the art and for example disclosed in WO200977575 and the references cited therein.

Within the scope of the present invention, the term " Magnetic Resonance investigation techniques", includes within its meaning any diagnostic technique which comprises administration of a (hyper)polarized substrate and the detection of MR signals of said substrate or of a metabolite thereof as commonly intended in the state of the art, including *in vivo* imaging metabolic activity, as disclosed for example in WO200977575 and the references cited therein.

Within the meaning of the present invention, the term "substrate" includes any compound enriched with non-zero nuclear spin nuclei (such as ¹H, ¹³C, ¹⁹F and/or ¹⁵N nuclei) which, when submitted to a DNP process, is capable of enhancing the nuclear polarization of its NMR active nuclei, i.e. "polarizes". The polarized substrate is in particular suitable for use in subsequent MR investigation techniques; preferably the substrate, e.g. when administered *in vivo,* is capable of being converted into respective polarized metabolic compound(s).

As used herein, the term "solid", when referred in particular to the substrate, includes within its meanings substances which are typically solid at room temperature, i.e. between 20°C and 25°C.Within the meaning of the present invention, the term "milling" means the process of breaking down materials, in particular using a mill, more in particular a ball mill. "Grinding" is also herein used as a synonym of milling.

Within the meaning of the present invention, the terms "polarizing agent" and "paramagnetic agent" are used as synonyms.

Within the meaning of the present invention, the terms "polarized" and "hyperpolarized" are used as synonyms.

### Detailed description of the invention

The method of the invention can be used when the polarization of a solid substrate is required.

The method of the present invention can be applied to any solid substrate having a crystalline form.

Preferably, it is applied to crystalline substrates stabilized by weak chemical bonds, such as Van der Waals, polar and/or H-bond interactions.

In a preferred embodiment, said substrate is an organic molecule having a crystalline form stabilized by polar and/or H-bond interactions.

In a more preferred embodiment, said substrate is selected from the group consisting of: amino acids, carbohydrates, hydroxy-acids, dicarboxylic acids and ketoacids.

When said substrate is an amino acid, it is preferably selected from arginine and glutamine. When said substrate is a carbohydrate, it is preferably selected from glucose, lactose and trehalose. When said substrate is an hydroxy-acid, it is preferably selected from lactic acid and malic acid. When said substrate is a dicarboxylic acid, it is preferably succinic acid.

Preferred solid compounds to be used as substrates are D-(+)-Glucose, α-Lactose and Trehalose β-polymorph.

The method of the invention may also be used for milling more than one solid substrate with the radical(s), in order to obtain a mixture of solid substrates and radical(s) to use as a single sample for DNP.

This is particularly useful when a single formulation containing a cocktail of substrates is desired, for example to simultaneously probe the activity of different enzymes.

For example, a mixture of crystalline lactose and crystalline glucose can be milled together with the selected radical according to the method of the invention to obtain a single amorphous sample to polarize.

According to the method of the invention, the solid substrate is milled together with a polarizing agent. Said polarizing agent is preferably a paramagnetic organic free radical. The polarizing agent is preferably solid at room temperature.

Examples of suitable polarizing agents are disclosed, for instance, in US 6,311,086 and include triarylmethyl radicals, nitroxide radicals, nitrogen centred radicals, oxygen centred radicals, stable carbon centred radicals and mixtures thereof.

Preferred organic free radicals are nitroxide radicals (such as porphyrexide, TEMPO, TEMPONE and TEMPOL), and stable carbon centered radicals (such as trityls or allyls radicals).

Examples of preferred radical compounds are 2,2,6,6,-Tetramethyl-1-piperidinyloxy (TEMPO), TEMPONE, TEMPOL, (tris(8-carboxy-2,2,6,6-(tetra(hydroxyethyl)-benzo-[1,2-4,5']-bis-(1,3)-dithiole-4-yl)-methyl sodium salt or tris(8-carboxyl-2,2,6,6-tetramethyl-benzo(1,2-d:4,5-dS)bis(1,3)dithiole-4-yl)methyl sodium salt and 1,3-bisdiphenylene-2-phenylallyl (BDPA).

A particularly preferred radical is TEMPO.

The polarizing agent concentration on the total mixture weight can be up to 2% (w/w); preferably it is from 0.1% to 1.5%, more preferably from 0.2% to 0.8% (w/w).

Preferably, the total volume of the mixture comprising the substrate and the radical, typically contained in a milling jar of a total volume of 45 ml, is of from 0.4 to 35 ml, more preferably from 0.6 to 15 ml and even more preferably from 0.7 to 3 ml. This allows to obtain an optimal homogeneous amorphization and a good mixing of the sample and the radical.

When the substrate is a crystalline solid, the polarizing agent may be admixed together with the substrate at any time during the milling process, e.g. before, during or at the end of the milling process (i.e. when the sample has reached the desired amorphous state). In this latter case, the amorphized sample can optionally be stored as such before admixing it with the polarizing agent. The polarizing agent is then admixed with the amorphized sample and the mixture is submitted to a co-milling process, in order to reach the desired intimate and homogeneous dispersion of the polarizing agent into the amorphous sample. Optionally, a partially amorphous substrate can be first obtained, which is then admixed with the polarizing agent and the mixture is then submitted to co-milling in order to obtain the desired amorphous sample with the polarizing agent intimately and uniformly dispersed therein. According to the invention, the solid substrate in a crystalline form is submitted to a milling process in the presence of a polarizing agent.

According to a preferred embodiment, the polarizing agent is first admixed with the substrate and the mixture is then submitted to a co-milling process.

As already explained above, the method of the invention can be carried out at room temperature for those substrates having a glass-transition temperature (Tg) above room temperature (preferably at least 10°C higher than room temperature, more preferably at least 30°C higher and even more preferably at least 50°C higher than RT).

For substrates having a Tg close to or lower than RT, the mill is preferably equipped with a cooling system, in order to perform the milling process at the required temperature lower than said Tg, preferably of at least 10°C lower than Tg, more preferably of at least 30°C lower than the Tg and even more preferably 50°C lower than the Tg.

The glass transition of a material is a pseudo second-order phase transition occurring during a cooling process of a melt or liquid-like state (undercooled liquid) that becomes a glass (amorphous solid), provided that the cooling rate is fast enough that crystallization is avoided. The glass transition may typically be observed through calorimetric or thermal analysis techniques in the cooling mode as well as, once the glass is formed, in the heating mode from glass to undercooled liquid. The glass transition temperature of a material (Tg) may vary slightly depending from the choice of the measurement technique and from the applied parameters (e.g. heating rate in Differential Scanning Calorimetry (DSC)). In accordance with common practice, where not otherwise specified herein, in the present specification and claims the glass transition is determined by DSC with heating scanning rate of 5°C/min and the value of Tg is taken as the midpoint of the quasi-sigmoidal portion of the DSC curve corresponding to the increase of specific heat (Cₚ) occurring at the glass transition.

Milling can be performed with any suitable instrument providing sufficient mechanical energy to disrupt the crystalline lattice of the substrate.

Preferably, the milling is performed using a ball mill, more preferably a planetary ball mill, even more preferably a high-energy planetary ball mill.

As an example, US6126097 discloses a high-energy planetary ball milling apparatus and protocol suitable for carrying out the method of the invention.

A suitable commercially available high-energy planetary ball milling apparatus is, for instance, Pulverisette 7 classic line (Fritsch, Germany).

In an embodiment of the invention, when the substrate is in crystalline form, milling is performed for a time suitable to obtain a sample in the amorphous state. Preferably, it is performed for a time comprised between 1 hour and 24 hour. More preferably it is performed for a time comprised between 6 hours and 12 hours. The suitable time can be chosen by the skilled in the art depending on the compound to be milled and on the temperature at which milling is performed. These milling times are generally sufficient to allow an intimate an homogeneous mixing of the polarizing agent admixed with the substrate.

In an aspect of the disclosure, particularly when the substrate is an amorphous or (partially) amorphized solid (e.g. obtained from a first milling step), milling is performed for a time suitable to obtain a homogeneous dispersion of the radical into the solid substrate. Preferably, it is performed for a time ranging between 10 minutes and 24 hours. More preferably it is performed for a time ranging between 1 hour and 6 hours.

While the above parameters and ranges are provided with respect to laboratory or pilot scale processes, based on the general knowledge and on the specific disclosure of the present invention, the skilled person is able to easily adapt these parameters and ranges to respective semi-industrial or industrial scale processes.

The achievement of an amorphous state - i.e. the substantial absence of crystalline phases in the sample - can be determined by conventional techniques such as, for instance, Raman spectroscopy and DSC. A Raman spectrum characteristic of a crystalline state presents many narrow and intense peaks (9), while a spectrum characteristic of an amorphous state presents very few broad and less intense bands. A calorimetric thermogram characteristic of a crystalline state presents only one (for one molecular species) exothermic narrow peak at the melting temperature (Tₘ); on the other hand, the thermogram of an amorphous state presents an exothermic broad band of absorbed water evaporation at T < 100°C (for scan rate = 5°C/min), a quasi-sigmoidal change in the specific heat Cₚ at the glass transition temperature Tg (transition from glass to "undercooled liquid"), an endothermic peak underlining the re-crystallization and an exothermic peak at Tₘ underlining the melting of the recrystallized phase.

As previously mentioned, the present process not only allows obtaining the substrate in the desired amorphous form but also allows an intimate and uniform dispersion of the polarizing agent (in particular of the radical) in the amorphous matrix of the sample.

In view of the above, the method of the invention provides a sample particularly suitable for an efficient polarization.

The obtained sample has also the advantage of remaining stable in the amorphous state for a relatively long period of time.

Therefore, it can be stored in order to preserve the amorphous state over sufficiently long time periods. This is particularly important for use of the amorphous sample for pharmaceutical applications.

To preserve the amorphous state, the obtained sample is preferably be maintained below its glass-transition temperature, preferably at a temperature of 10°C lower than the Tg, more preferably at a temperature of 50°C lower than its Tg.

Moreover, it can be preferably maintained in a controlled atmosphere, in particular in low humidity conditions, to further preserve its stability over longer periods of time.

For instance, the milled sample may remain stable (i.e. substantially amorphous) for at least 20 days under air or for at least 72 days under inert atmosphere (e.g. argon, O₂ and H₂O content < 0.1 ppm).

The amorphous sample obtained by milling the substrate with the radical according to the method of the invention can then be hyperpolarized according to standard dynamic nuclear polarization protocols.

Reference can be made for example to WO98/58272 or WO2011124672.

An efficient DNP process is usually performed at high magnetic field (for example 3-8 T) and low temperatures (for example 0.5-5 K).

A polarized (DNP) sample comprising the polarized substrate of interest is thus obtained.

After the DNP process, the sample can be dissolved in an appropriate solvent, for example water, or solvent mixtures in order to obtain a solution for Magnetic Resonance Imaging (MRI) analysis.

A further aspect of the invention relates therefore to a method for preparing a solution for MRI analysis which comprises the steps of:
a) preparing a solid polarized sample as above described, and
b) dissolving the solid polarized sample in an appropriate solvent.

Dissolution of the sample is performed by addition of a suitable dissolving medium, preferably a physiologically tolerable aqueous carrier. Preferably the dissolution is performed in a relatively short time (few seconds) while the sample is maintained in the high magnetic field, for instance by admixture with hot aqueous carrier. Details on the dissolution process of a frozen hyperpolarised mixture and suitable devices for performing the dissolution are provided, for instance, in WO-A-02/37132.

Substances commonly used to regulate the pH at physiological values (e.g. HCI, NaOH, buffers) can also be added to the solution to obtain a sample suitable for injection in a subject. The appropriate substances should be chosen according to the component(s) of the solid sample and this can be done by the skilled person referring to its general knowledge in the field.

The radical and or reaction products thereof may be removed from the dissolved polarized compound. Methods usable to partially, substantially or completely remove the radical and/or reaction products thereof are known in the art. Generally, the methods applicable depend on the nature of the radical and/or its reaction products. For example, upon dissolution of the solid hyperpolarized compound, the radical might precipitate and then be easily separated from the liquid by filtration. If no precipitation occurs, the radical may be removed by chromatographic separation techniques, e.g. liquid phase chromatography like reversed phase, straight phase or ion exchange chromatography or by extraction. Alternatively, free radicals can be eliminated by adding a compound acting as a "scavenger" (for example a reducing agent like sodium ascorbate) to the mixture of substrate with radical so that when the mixture is dissolved said "scavenger" neutralizes the radical (see for reference US8564288B2).

The solution containing the hyperpolarized sample obtained with the method of the invention can thus be used as an MRI contrast agent for both *in vitro* and *in vivo* applications.

For example, it can be used *in vitro* on isolated biological samples or on cell cultures or administered to an animal or human subject for *in vivo* applications. Typically the solution containing the hyperpolarized sample is administered (e.g. by injection) within few seconds from the preparation thereof. In general, optimal concentrations will in most cases lie in the range from 10 mM to 250 mM, particularly from 150 to 250 mM. In any case, the dosage of the solution should be kept as low as possible whilst still providing a detectable signal of the polarized substrate or metabolites thereof.

The MR signals detected from the hyperpolarized substrate(s) or metabolites thereof can be used to generate an image, physiological data or metabolic data.

Indeed, it is a further object of the present disclosure to provide a method of MR analysis comprising administering to a subject a solution for MR analysis comprising an hyperpolarized sample prepared as above described and detecting the resulting MR signal.

MR analysis can be performed according to standard protocols known in the art.

The method of the invention is preferably used for obtaining hyperpolarized samples of organic substrates which can be used as tracers for metabolic applications, for example to follow the metabolic conversion of a certain organic compound or for the detection of metabolic abnormalities associated with diseases.

The present invention will be further illustrated by the following examples.

### EXAMPLES

Where not otherwise specified, chemicals and reagents used in the following examples are commercially available or can be prepared according to methods well-known in the art.

### Materials

The following materials have been employed in the subsequent examples:
- commercial crystalline TEMPO (2,2,6,6,-Tetramethyl-1-piperidinyloxy, from Sigma Aldrich) as solid radical component;
- commercial anhydrous crystalline β-Trehalose (from Acros Organics);
- commercial anhydrous crystalline α-Lactose (from Acros Organics);
- commercial crystalline D-(+)-Glucose (from Sigma-Aldrich);
- commercial crystalline Fumaric Acid (from Sigma-Aldrich).

### Methods

The sample preparation is made by admixing the substrate to be polarized with the polarizing agent (TEMPO, 2,2,6,6,-Tetramethyl-1-piperidinyloxy) and submitting the mixture to milling by using a high energy planetary micro mill (Pulverisette 7 classic line from Fritsch, Germany). Each sample has been inserted in a ZrO₂ milling bowl (volume about 45 cm³) containing seven ZrO₂ balls (diameter 15 mm) and the rotation frequency of the solar disk has been set at 400 rpm. A homogeneous amorphization and a good mixing of the two species is reached by using a total mass of at least 1.1 g and a mixing time of at least 12 hrs (7, 8, see also US6126097). The process temperature was kept always lower than the Tg (T_{process} < T_{g} of 50°C); pauses in the process were inserted to avoid raising in temperature (5 minutes every 15 minutes of milling at 400rpm).

The achievement of an amorphous state - i.e. the absence of crystalline phases - was determined by Raman spectroscopy and DSC. A Raman spectrum characteristic of a crystalline state presents many narrow and intense peaks (9), while that characteristic of an amorphous state presents very few broad and less intense bands. A calorimetric thermogram characteristic of a crystalline state presents only one (for one molecular species) exothermic narrow peak at the melting temperature (Tₘ); on the other hand, the thermogram of an amorphous state presents an exothermic broad band of absorbed water evaporation at T < 100°C (for scan rate = 5°C/min), a quasi-sigmoidal change in Cₚ at the glass transition temperature T_{g} (transition from glass to "undercooled liquid"), an endothermic peak underlining the re-crystallization and an exothermic peak at Tₘ underlining the melting of the recrystallized phase.

Aliquots of milled sample have been used for the characterization of the structure and the analysis of the polarization properties:
- about 100 mg for Raman Spectrometric measurements;
- about 5-10 mg for Differential Scanning Calorimetric measurements;
- about 5 mg for Thermogravimetric measurements;
- about 65 to 85 mg for Dynamic Nuclear Polarization measurements.

### Example 1

### Preparation and characterization of a milled Trehalose sample for DNP

### Raman and Calorimetric measurements on a crystalline (β) Trehalose

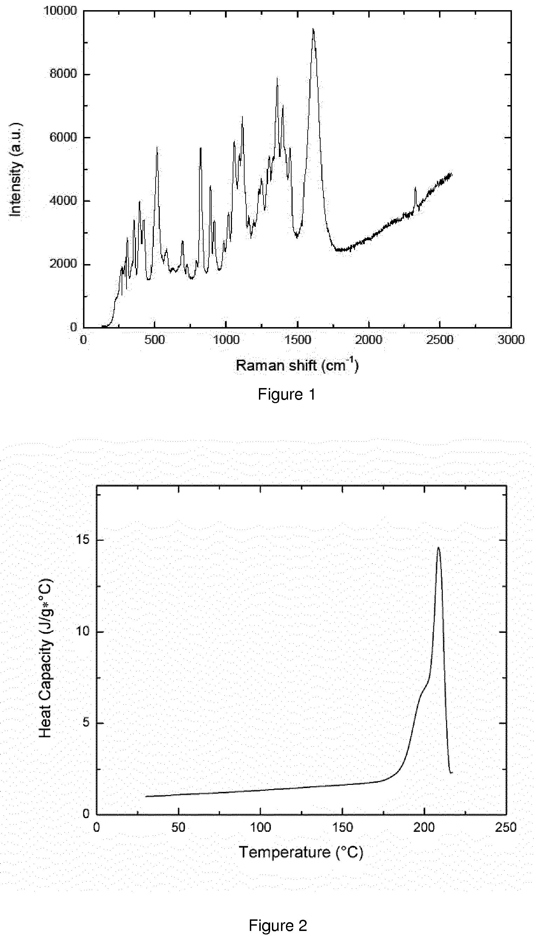

Two aliquots of crystalline β-Trehalose have been analyzed respectively by means of Raman Spectroscopy and Differential Scanning Calorimetry, giving the results in Figures 1 and 2. The Raman spectrum is recorded at room temperature and is characteristic of a highly crystalline material. The thermogram shows no other peak but the melting of the crystalline material starting at about 185°C.

### Preparation and characterization of milled Trehalose sample for DNP

A mixture of crystalline Trehalose and crystalline TEMPO has been co-milled for 12 h at room temperature. In particular samples with radical concentration in the mixture of 0.15, 0.34, 0.50, 0.64 and 0.81 % (w/w) have been prepared.

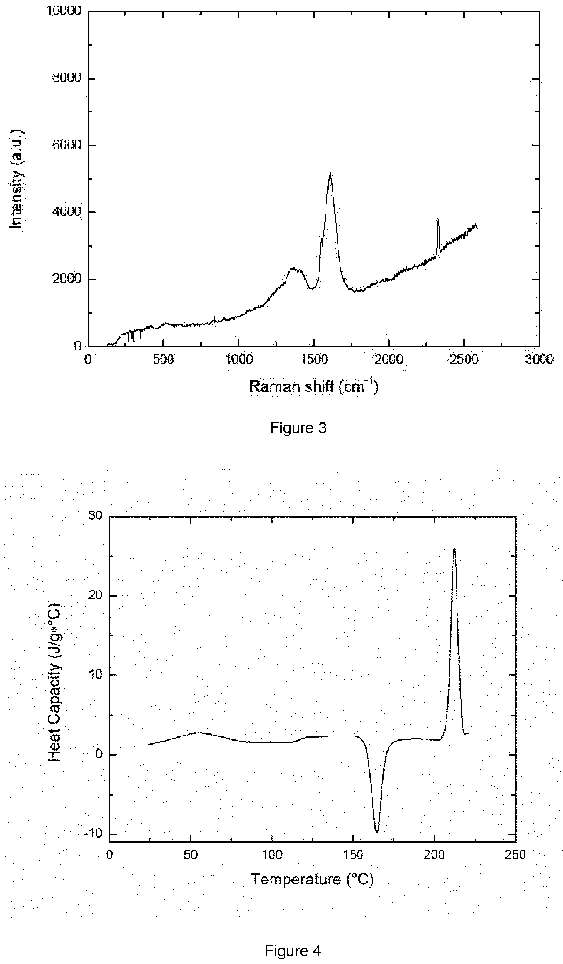

The milled sample with 0.50 % of TEMPO has been analysed by means of Raman Spectroscopy and Differential Scanning Calorimetry, giving the results in Figures 3 and 4. The Raman spectrum appears to be devoid of the characteristic peaks of the crystalline phase, and there is a glass transition temperature around 120°C, characteristic of the disordered state of the Trehalose matrix, which thereafter undergoes cold-crystallization (negative peak in the DSC diagram of figure 4) and subsequent melting upon further heating (positive peak around 215°C in figure 4). A small amount of water evaporates at T < 80 °C.

### Example 2

### Preparation and characterization of a milled Lactose sample for DNP

A mixture of crystalline Lactose and crystalline TEMPO has been co-milled for 12 h at room temperature; the radical concentration in the mixture is 0.48 %(w/w).

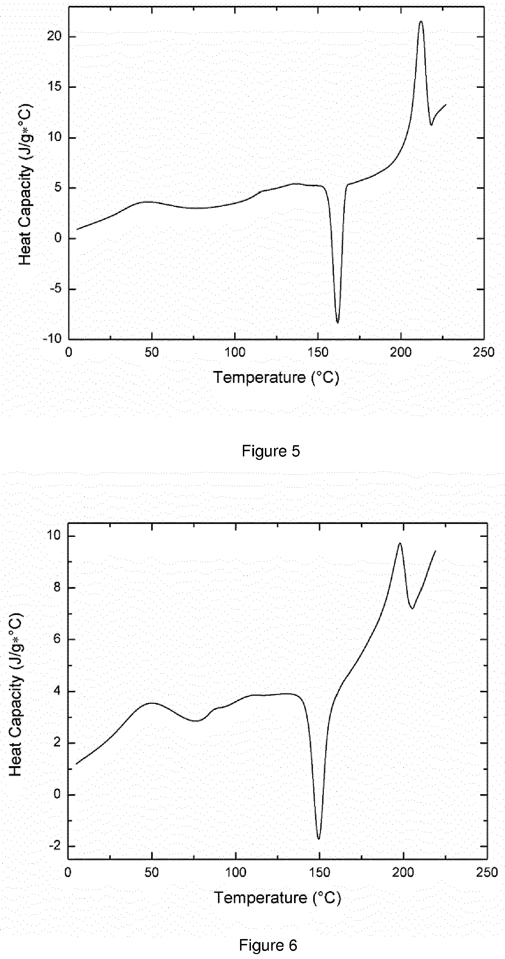

The resulting sample, analyzed by means of Differential Scanning Calorimetry (Figure 5), presents a glass transition temperature around 110 °C, characteristic of the disordered state of the Lactose matrix, which thereafter undergoes cold-crystallization and subsequent melting upon further heating.

### Example 3

### Preparation and characterization of a co-milled Lactose-Glucose sample for DNP

A mixture of crystalline Lactose, crystalline Glucose and crystalline TEMPO has been co-milled for 12 h at 12 °C; the radical concentration in the mixture is 0.60 %(w/w) while the ratio between the Glucose and Lactose masses is 1:4.

The resulting sample, analyzed by means of Differential Scanning Calorimetry (Figure 6), presents a glass transition occurring at around 80 °C consistent with a disordered phase composed by Lactose and Glucose.

### Example 4

### Preparation of a milled Fumaric Acid sample for DNP

A mixture of crystalline Fumaric Acid and crystalline TEMPO has been co-milled for 12 h at 12 °C; the radical concentration in the mixture is 0.49% (w/w).

### Example 5

### Thermogravimetric measurements on milled Lactose

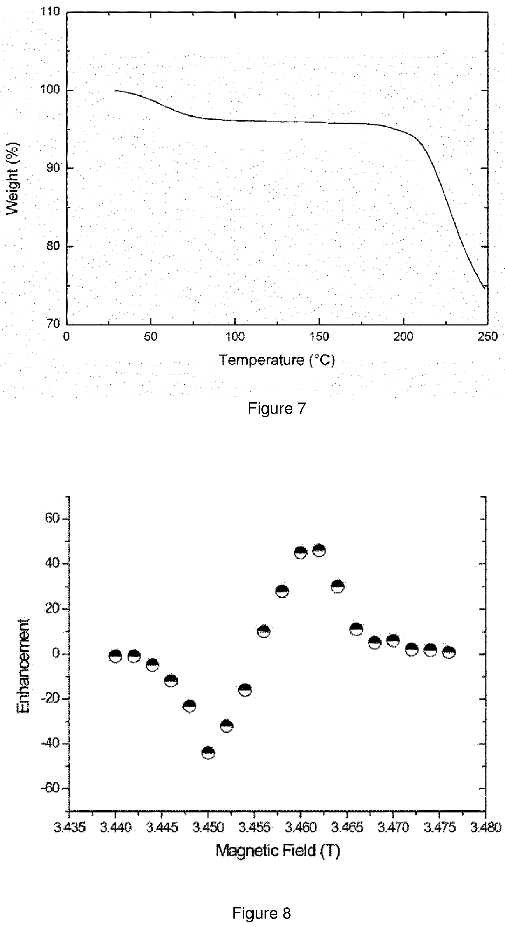

An aliquot of the milled sample of Lactose (see Example 2) has been analyzed during the 2nd day after the amorphization by means of thermogravimetry. Figure 7 shows the sample weight as function of the temperature, enhancing a 4% weight loss up to 70 °C due to the evaporation of water adsorbed during the milling process and the sample storage; the other weight loss above 200 °C is due to the degradation of the Lactose.

Similar behaviour is observed for the other milled samples composed by sugars, while the milled Fumaric Acid is expected to contain a smaller amount of water.

### Example 6

### DNP enhancement of milled Trehalose as a function of the magnetic field

An aliquot of the milled sample of Trehalose with radical concentration of 0.64 % prepared according to Example 1 has been transferred into a quartz tube inside a glove box at room temperature and the tube has been capped by using Teflon tape. The sample has been cooled in a bath cryostat at 1.7 K and irradiated with MicroWaves (MW) at 96.93 GHz with a 30 mW power source. The 1H NMR signal enhancement (ε, i.e. the ratio between the steady state signal intensity under MW irradiation and the steady state signal intensity at thermal equilibrium) has been measured upon varying the applied magnetic field H between 3.44 T and 3.475 T. The behaviour of the polarization enhancement (ε) versus the applied magnetic field (H) has a shape that recalls the derivative of the EPR line of the TEMPO radical as a function of the magnetic field (Fig. 8). In the literature this behaviour has been considered as a fingerprint of dynamic nuclear polarization occurring via thermal mixing. The maximum value of ε measured in the milled sample of Trehalose with radical concentration of 0.64 % is about 50 corresponding to a nuclear spin polarization (P) of 10 %. This is approximately half of the one obtained with the same system at 3.46 T in a 3 M solution of Sodium Acetate (NaAc) in D₂O (67%) and deuterated ethanol (33%), containing TEMPO with a concentration of 30 mM.

### Example 7

### DNP enhancement of milled Trehalose as a function of radical concentration

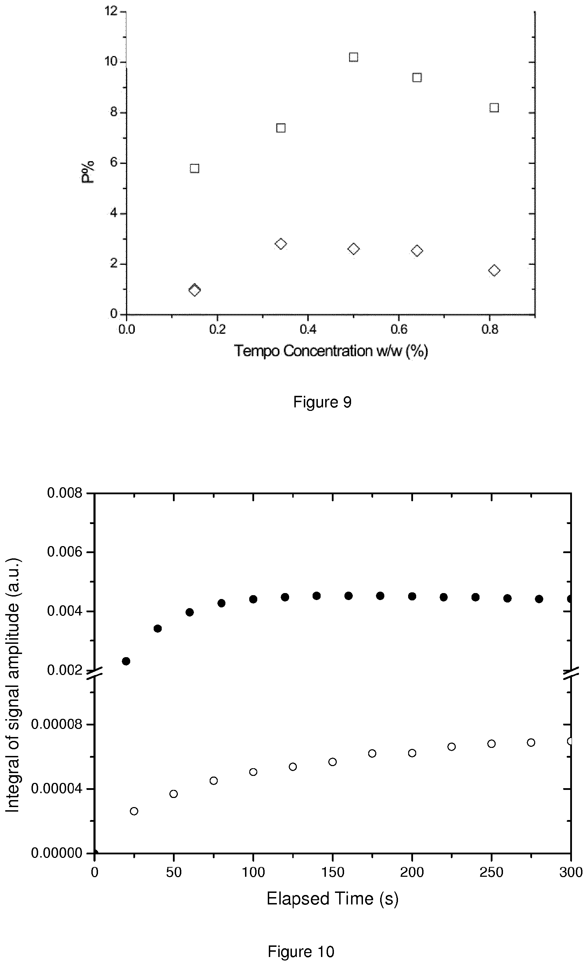

The MW irradiation frequency was fixed to the 96.93 MHz to maximize the positive enhancement of the NaAc reference at 3.46 T. The milled samples of Trehalose (see Example 1) were transferred in quartz tubes (the procedure is described in the Example 6). Then ε was measured in all the samples between 4.2 K and 1.7 K. The data in figure 9 show an increase of P up to 10% at 1.75 K for a concentration of 0.5-0.6% of TEMPO and then a slow decrease of P at higher concentrations (squares in fig. 9). A similar trend is observed at 4.2 K (rhombi in fig. 9), but the maximum of P is attained at lower TEMPO concentration, around 0.3-0.5%.

### Example 8

### Maximum achievable DNP polarization (P) of several milled samples

On the first day after the amorphization, 50-70 mg of the milled sample of Trehalose and TEMPO (0.64%) and of the samples described in the Examples 2-4 were transferred in quartz tubes according to the procedure described in Example 6. A tube containing Lactose (sample 1) was immediately inserted in the helium bath for DNP measurements while the other tubes were stored in the freezer for subsequent measurements on days indicated in Table 1. All the DNP measurements were performed at the frequency maximizing positive polarization in NaAc at 3.46 T. The polarization build up was measured at 1.6-1.7 K resorting to small angle acquisition schemes of the ¹H NMR signal. By way of example, in Fig. 10 the polarization build-up curve in milled Lactose+Glucose with radical concentration of 0.60% obtained under MW irradiation (full dots) is shown together with a spontaneous (i.e. with MW off) recovery towards thermal equilibrium of the same sample. The summary of the measurement performed in all the samples at 1.6-1.7 K on days 1st-8th after the amorphization is reported in Table 1. All samples reached significantly high P levels with respect to the thermal one, equal to 0.21%. The highest value of P (12%) is observed in the mixture of Lactose and Glucose (Example 3).

**Table 1: Polarization level of ¹H (1.6-1.7 K and 3.46 T) in the samples of Ex. 1-4**

| **Example** | **P% *** | **Days after amorphization** | **Storage** |
|---|---|---|---|
| 1 | 10 | 8 | freezer |
| 2 | 7.5 | 1 | - |
| 3 | 12 | 7 | freezer |
| 4 | 0.83 | 3 | freezer |

| | | | |
|---|---|---|---|
| * to be compared with thermal equilibrium P (0.21%). | | | |

### Example 9

### Physical stability of milled Lactose

After extraction from the cryogenic bath a first aliquot of the Lactose sample of Example 8 (Lactose - sample 1) was stored in a glove box (argon atmosphere, O₂ and H₂O content < 0.1 ppm). An equivalent aliquot of the same sample (Lactose-sample 2) was left in the same room but outside the glove box. DNP measurements on the two samples of Lactose were then repeated after three weeks and after two months of ageing. The summary of the results obtained at 1.6-1.7 K is reported in table 2.

**Table 2: Polarization level of ¹H (1.6-1.7 K and 3.46 T) in DNP lactose samples upon ageing**

| **Samples at 1.7 K** | **P% *** | **Days after amorphization** | **Storage** |
|---|---|---|---|
| Lactose - sample 1 | 7.5 | 1 | 20°C - inside GB |
| Lactose - sample 1 | 7.5 | 20 | 20°C - inside GB |
| Lactose - sample 2 | 8.7 | 21 | 20°C - outside GB |
| Lactose - sample 1 | 8.9 | 72 | 20°C - inside GB |
| Lactose - sample 2 | 0.8 | 73 | 20°C - outside GB |

| | | | |
|---|---|---|---|
| GB= Glove Box; ***** to be compared with thermal equilibrium (0.21%). | | | |

As illustrated by the above table the polarization level of achievable on the aged samples inside or outside the glove box remains substantially constant after three weeks from the milling of the crystalline substrate, while after 2 months the sample kept out of the glove box shows a decrease of the polarization level.

### Example 10

### Rapid dissolution of a Lactose sample to achieve a ready-to-inject solution

85 mg (about 0.25 mmol) of Lactose-TEMPO solid solution, prepared by co-milling as described in Example 2, is flashed frozen in a He-bath and polarized under standard DNP conditions (magnetic field 3.46 T, MW frequency 96.93 GHz, temperature 1.75 K) for 1 hour to allow both ¹H and ¹³C nuclei to get to a hyperpolarized steady state, corresponding for the former nuclei to a polarization level of about 8%. At the end of the DNP procedure, the MW irradiation is switched off, the sample is raised 10 cm above the center of the magnetic field and dissolved by 5 ml of overheated water (about 10 bar, 170 °C) to obtain a liquid solution with 50 mM Lactose concentration, suitable for use in animal experiments (0.4 mmol/kg of Lactose for administered doses of 8 ml/kg).

### References

1. Measurements of glass properties and density of hydrocarbon mixtures of interest in polarized targets. CERN, CH-1211, 347-352.
2. Bunyatova EI. New investigation of organic compounds for targets with polarized hydrogen nuclei. Nucl Instrum Meth Phys Res, Sect A 1995; 356: 29-33.
3. Increase in signal-to-noise ratio of >10,000 times in liquid-state NMR. PNAS 2003; 100(18): 10158-10163.
4. Design and Performance of a DNP Prepolarizer Coupled to a Rodent MRI Scanner. Concepts in Magnetic Resonance Part B: Magnetic Resonance Engineering 2007; 31B(4): 255-269.
5. Development of Dissolution DNP-MR Substrates for Metabolic Research. Applied Magnetic Resonance 2012; 43(1-2): 223-236.
6. Solvent-Free Dynamic Nuclear Polarization of Amorphous and Crystalline ortho-Terphenyl. J Phys Chem B. Mar 14, 2013; 117(10): 3040-3046.
7. Solid state amorphization kinetic of alpha lactose upon mechanical milling. Carbohydr. Res. 2011; 346(16): 2622-2628.
8. Direct crystal to glass transformation of trehalose induced by ball milling. Solid State Commun. 2001; 119: 501-505.
9. Spectroscopic studies of solid α-α Trehalose. Spectrochimica Acta Part A: Molecular and Biomolecular Spectroscopy 1996; 52(12); 1649-1659.

## Claims

1. A method for the manufacture of a sample in amorphous form for dynamic nuclear polarization which comprises submitting a solid substrate in a crystalline form to a milling process in the presence of a polarizing agent at a temperature lower than the glass-transition temperature (Tg) of said substrate.

2. The method according to claim 1 wherein said substrate is selected from the group consisting of: amino acids, carbohydrates, hydroxy-acids, dicarboxylic acids and ketoacids.

3. The method according to anyone of claims 1 or 2 wherein said substrate is selected from the group consisting of arginine, glutamine, glucose, lactose, trehalose, lactic acid, malic acid and succinic acid.

4. The method according to anyone of claims 1 to 3 wherein said substrate is selected from the group consisting of D-(+)-Glucose, α-Lactose and Trehalose β-polymorph.

5. The method according to anyone of claims 1 to 4 wherein two or more substrates are in solid crystalline form and they are mixed with the at least one polarizing agent.

6. The method according to anyone of claims 1 to 5 wherein said at least one polarizing agent is an organic free radical, preferably selected from triarylmethyl, nitroxide radicals, nitrogen centred radicals, oxygen centred radicals, stable carbon centred radicals and mixtures thereof.

7. The method according to claim 6 wherein said radical is selected from the group consisting of porphyrexide, TEMPO, TEMPONE, TEMPOL, tris(8-carboxy-2,2,6,6-(tetra(hydroxyethyl)-benzo-[1,2-4,5']-bis-(1,3)-dithiole-4-yl)-methyl sodium salt, tris(8-carboxyl-2,2,6,6-tetramethyl-benzo(1,2-d:4,5-dS)bis(1,3)dithiole-4-yl)methyl sodium salt and 1,3-bisdiphenylene-2-phenylallyl (BDPA).

8. The method according to anyone of claims 1 to 7 wherein the polarizing agent concentration on the total mixture weight is comprised between 0.1% and 1.5%, preferably between 0.2% and 0.8% (w/w).

9. The method according to anyone of the preceding claims wherein said milling is performed at a temperature at least 10°C lower than the glass-transition temperature of said substrate, preferably at a temperature at least 30°C lower and more preferably at a temperature at least 50°C lower.

10. The method according to anyone of the preceding claims wherein said at least one substrate has a glass-transition temperature (Tg) above room temperature and said milling is performed at room temperature.

11. The method according to anyone of the preceding claims wherein said milling is performed using a planetary ball mill.

12. A method for preparing a polarized sample comprising a substrate polarized by dynamic nuclear polarization (DNP) which comprises the following steps:
a) submitting a solid substrate in a crystalline form to a milling process in the presence of a polarizing agent according to the method of anyone of claims 1-11 thus obtaining a sample for DNP in amorphous form;
b) submitting said sample to DNP to obtain a polarized sample comprising said polarized substrate.

13. A method for preparing a solution for MR analysis comprising the following steps:
a) preparing a polarized sample using the method of claim 12, and
b) dissolving said polarized sample in an appropriate solvent.

## Patentansprüche

1. Verfahren zur Herstellung einer Probe in amorpher Form für die dynamische Kernpolarisation, dass das Unterziehen eines festen Substrats in einer kristallinen Form einem Mahlprozess in Gegenwart eines Polarisierungsmittels bei einer Temperatur unterhalb der Glasübergangstemperatur (Tg) des Substrats umfasst.

2. Verfahren nach Anspruch 1, wobei das Substrat aus der Gruppe ausgewählt wird, die besteht aus: Aminosäuren, Kohlenhydraten, Hydroxysäuren, Dicarbonsäuren und Ketosäuren.

3. Verfahren nach einem der Ansprüche 1 oder 2, wobei das Substrat ausgewählt wird aus der Gruppe, die aus Arginin, Glutamin, Glukose, Laktose, Trehalose, Milchsäure, Apfelsäure und Bernsteinsäure besteht.

4. Verfahren nach einem der Ansprüche 1 bis 3, wobei das Substrat ausgewählt wird aus der Gruppe, die aus D-(+)-Glukose, a-Laktose und Trehalose β-polymorph besteht.

5. Verfahren nach einem der Ansprüche 1 bis 4, wobei zwei oder mehr Substrate in fester kristalliner Form sind, und sie werden mit dem mindestens einen Polarisierungsmittel gemischt.

6. Verfahren nach einem der Ansprüche 1 bis 5, wobei das mindestens eine Polarisierungsmittel ein organisches freies Radikal ist, das vorzugsweise aus Triarylmethyl-, Nitroxid-Radikalen, stickstoff-zentrierten Radikalen, sauerstoff-zentrierten Radikalen, stabilen kohlenstoffzentrierten Radikalen und Mischungen derselben besteht.

7. Verfahren nach Anspruch 6, wobei das Radikal ausgewählt wird aus der Gruppe, die aus Porphyrexid, TEMPO, TEMPONE, TEMPOL, Tris(8-carboxy-2,2,6,6-(tetra(hydroxyethyl)-benzo-[1,2-4,5']-bis-(1,3)-dithiol-4-yl)-methyl-Natriumsalz, Tris(8-carboxyl-2,2,6,6-tetramethyl-benzo(1,2-d:4,5-dS)bis(1,3)dithiol-4-yl)-methyl-Natriumsalz und 1,3-Bisdiphenylen-2-phenylallyl (BDPA) besteht.

8. Verfahren nach einem der Ansprüche 1 bis 7, wobei die Konzentration des Polarisierungsmittels, bezogen auf das Gesamtmischungsgewicht, zwischen 0,1 % und 1,5 % liegt, vorzugsweise zwischen 0,2 % und 0,8 % (w/w).

9. Verfahren nach einem der vorherigen Ansprüche, wobei das Mahlen bei einer Temperatur von mindestens 10 °C niedriger als die Glasübergangstemperatur des Substrats ausgeführt wird, vorzugsweise bei einer Temperatur von mindestens 30 °C tiefer und noch besser bei einer Temperatur von mindestens 50 °C tiefer.

10. Verfahren nach einem der vorherigen Ansprüche, wobei das mindestens eine Substrat eine Glasübergangstemperatur (Tg) nicht oberhalb der Raumtemperatur hat, und das Mahlen wird bei Raumtemperatur ausgeführt.

11. Verfahren nach einem der vorherigen Ansprüche, wobei das Mahlen unter Verwendung einer Planetenkugelmühle ausgeführt wird.

12. Verfahren zum Herstellen einer polarisierten Probe, die ein Substrat enthält, das durch dynamische Kernpolarisation (DNP) polarisiert wird, das die folgenden Schritte umfasst:
a) Unterziehen eines festen Substrats in kristalliner Form einem Mahlprozess in Anwesenheit eines Polarisierungsmittels entsprechend dem Verfahren nach einem der Ansprüche 1 bis 11, wodurch eine Probe für DNP in amorpher Form erhalten wird;
b) Unterziehen der Probe der DNP, um eine polarisierte Probe zu erhalten, die das polarisierte Substrat enthält.

13. Verfahren zum Herstellen einer Lösung zur MR-Analyse, das die folgenden Schritte umfasst:
a) Herstellen einer polarisierten Probe unter Verwendung des Verfahrens nach Anspruch 12, und
b) Auflösen der polarisierten Probe in einem geeigneten Lösungsmittel.

## Revendications

1. Procédé de fabrication d'un échantillon sous forme amorphe pour une polarisation dynamique nucléaire comprenant le fait de soumettre un substrat solide sous une forme cristalline à un procédé de broyage en présence d'un agent polarisant à une température inférieure à la température de transition vitreuse (Tg) dudit substrat.

2. Procédé selon la revendication 1, dans lequel ledit substrat est sélectionné dans le groupe constitué d'acides aminés, de glucides, d'hydroxyacides, d'acides dicarboxyliques et de cétoacides.

3. Procédé selon l'une quelconque des revendications 1 ou 2, dans lequel ledit substrat est sélectionné dans le groupe constitué de l'arginine, de la glutamine, du glucose, du lactose, du tréhalose, de l'acide lactique, de l'acide malique et de l'acide succinique.

4. Procédé selon l'une quelconque des revendications 1 à 3, dans lequel ledit substrat est sélectionné dans le groupe constitué du D-(+)-glucose, de l'a-lactose et du tréhalose β-polymorphe.

5. Procédé selon l'une quelconque des revendications 1 à 4, dans lequel deux substrats ou plus sont sous une forme cristalline solide et sont mélangés avec ledit au moins un agent polarisant.

6. Procédé selon l'une quelconque des revendications 1 à 5, dans lequel ledit au moins un agent polarisant est un radical libre organique, préférablement sélectionné parmi le triarylméthyle, des radicaux nitroxyde, des radicaux à centre azote, des radicaux à centre oxygène, des radicaux stables à centre carboné, et des mélanges de ceux-ci.

7. Procédé selon la revendication 6, dans lequel ledit radical est sélectionné dans le groupe constitué du porphyrexide, du TEMPO, du TEMPONE, du TEMPOL, du sel sodique de tris(8-carboxy-2,2,6,6-(tétra(hydroxyéthyl)-benzo-[1,2-4,5']-bis-(1,3)-dithiole-4-yl)-méthyle, du sel sodique de tris(8-carboxyl-2,2,6,6-tétraméthyl-benzo(1,2-d:4,5-dS)bis(1,3)dithiole-4-yl)méthyle et du 1,3-bisdiphénylène-2-phénylallyle (BDPA).

8. Procédé selon l'une quelconque des revendications 1 à 7, dans lequel la concentration de l'agent polarisant relativement au poids total du mélange est de 0,1 % à 1,5 %, préférablement de 0,2 % à 0,8 % (p/p).

9. Procédé selon l'une quelconque des revendications précédentes, dans lequel ledit broyage est réalisé à une température inférieure d'au moins 10 °C à la température de transition vitreuse dudit substrat, préférablement à une température inférieure d'au moins 30 °C et plus préférablement à une température inférieure d'au moins 50 °C.

10. Procédé selon l'une quelconque des revendications précédentes, dans lequel ledit au moins un substrat a une température de transition vitreuse (Tg) supérieure à la température ambiante et ledit broyage est réalisé à la température ambiante.

11. Procédé selon l'une quelconque des revendications précédentes, dans lequel ledit broyage est réalisé en utilisant un broyeur planétaire à boulets.

12. Procédé de préparation d'un échantillon polarisé comprenant un substrat polarisé par polarisation dynamique nucléaire (PDN), comprenant les étapes suivantes :
a) le fait de soumettre un substrat solide sous une forme cristalline à un procédé de broyage en présence d'un agent polarisant par le procédé selon l'une quelconque des revendications 1 à 11, en obtenant ainsi un échantillon pour la PDN sous une forme amorphe ;
b) le fait de soumettre ledit échantillon à la PDN pour obtenir un échantillon polarisé comprenant ledit substrat polarisé.

13. Procédé de préparation d'une solution pour l'analyse RM, comprenant les étapes suivantes :
a) la préparation d'un échantillon polarisé par le procédé selon la revendication 12, et
b) la dissolution dudit échantillon polarisé dans un solvant approprié.
